# EUROPEAN PATENT SPECIFICATION

(11) **EP 0 775 484 B1**
(45) Date of publication and mention of the grant of the patent: **03.09.2003**
(21) Application number: 97101101.0
(22) Date of filing: 30.07.1993
(51) Int. Cl.: A61K 9/00, A61K 31/57

(54) **Surfactant free aerosol formulations containing beclomethsone dipropionate**
Oberflächenaktivmittelfreie Aerosolformulierungen enthaltend Beclomethasondipropionat
Formulations d'aerosol sans tensioactif contenant du propionate de beclométhasone

(30) Priority: 31.07.1992 GB 9216381; 31.07.1992 GB 9216382
(43) Date of publication of application: 28.05.1997
(62) Divisional of application: 93917708.5
(73) Proprietor: GLAXO GROUP LIMITED, Greenford, Middlesex UB6 0NN (GB)
(72) Inventor: Taylor, Anthony James, Ware, Herts SG12 0DP (GB); Neale, Philip John, Ware, Herts SG12 0DP (GB)
(74) Representative: Quillin, Helen Kaye

(56) References cited:
- EP-A- 0 518 600
- WO-A-92/06675
- WO-A-92/22287
- Merck Index, 9th ed., 1025 beclomethasone

## Description

This invention relates to novel aerosol formulations for administering drugs, in particular for administration of a beclomethasone ester by inhalation.

Beclomethasone dipropionate is 9α-chloro-16β-methyl-1,4-pregnadiene -11β, 17α,21-triol-3,20-dione 17α,21-dipropionate and may be represented by the formula (I)

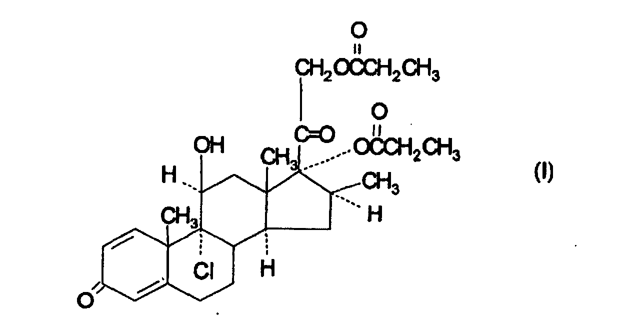

The corticosteroid of formula (I) is known to exhibit topical antiinflammatory activity and is useful in the treatment of asthmatic conditions, particularly in the form of aerosol formulations. The use of such formulations is described in GB-1429184 where it is noted that micronised anhydrous beclomethasone dipropionate tends to display crystal growth, due to solvate formation, when incorporated into aerosol formulations containing chlorofluorocarbon propellants. Crystals having a particle size of more than 20 microns were shown to be too large to penetrate the bronchial system and prone to cause clogging of the metering valve making them unsuitable for administration by inhalation.

A number of potential solutions to this problem have been proposed. These include the use of micronised solvates of beclomethasone dipropionate, for example chlorofluorocarbon solvates (GB-1429184), ethyl acetate solvate (DE-3018550OS), C₅₋₈alkane solvates (EP-0039369), diisopropyl ether solvate (EP-0172672) and C₁₋₅ alcohol solvates (WO86/03750). GB-2076422A discloses a process for the preparation of chlorofluorocarbon aerosols which incorporates a low temperature (5 to -40°C) step which is also claimed to inhibit crystal growth.

An alternative solution to the problem of crystal growth in aerosol formulations containing beclomethasone dipropionate has recently been disclosed in WO92/06675. This document describes the preparation of aerosol formulations containing solutions of beclomethasone diproprionate in ethanol, together with hydrofluorocarbon 134a (1,1,1,2-tetrafluoroethane) or hydrofluorocarbon 227 (1,1,1,2,3,3,3-heptafluoropropane) as propellant. Since a solution of beclomethasone dipropionate in ethanol is employed in the aerosols rather than a suspension of particulate beclomethasone diproprionate, elaborate process steps or the preparation of a solvate of the active ingredient prior to incorporation into the aerosol formulation is not required.

Nevertheless, whilst ethanol is pharmaceutically acceptable and generally recognised as safe, it is associated with a number of disadvantages which may restrict is use. In particular, administration of ethanol-containing products to teetotal or alcohol-dependent individuals or to children is undesirable.

EP-0518600, WO92/22287 and WO92/22288 describe other aerosol formulations using 1,1,1,2-tetrafluoroethane or 1,1,1,2,3,3,3-heptafluoropropane as a propellant.

A number of other patent applications describe the preparation of aerosol formulations containing drug and a fluorocarbon propellant, together with the addition of one or more adjuvants such as surfactants. Thus, for example WO91/14422 describes the preparation of aerosol formulations containing beclomethasone dipropionate in the form of its 1,1,1,2-tetrafluoroethane clathrate together with 1,1,1,2-tetrafluoroethane and various surface-active dispersing agents.

We have now found that certain novel aerosol formulations containing particulate beclomethasone dipropionate are surprisingly stable without recourse to the use of any adjuvant or cosolvent in the composition.

The present invention therefore provides a canister comprising an aluminium can optionally anodised, lacquer coated and/or plastic coated and closed with a metering valve which contains a pharmaceutical aerosol formulation which comprises particulate anhydrous beclomethasone dipropionate and 1,1,1,2,3,3,3-heptafluoro-n-propane as propellant, which formulation is substantially free of surfactant. By "substantially free of surfactant" is meant formulations which contain no significant amounts of surfactant, for example less than 0.0001% by weight of the beclomethasone dipropionate.

The particle size of the particulate beclomethasone dipropionate may be reduced by conventional methods, for example by micronisation, fluid energy milling or ball milling and should be such as to permit inhalation of substantially all of the drug into the lungs upon administration of the aerosol formulation. Preferably the particle size of the beclomethasone dipropionate will be less than 20 microns, most preferably less than 10 microns, in particular in the range of 1 to 5 microns.

It is desirable that the formulations of the invention contain no components which may provoke the degradation of stratospheric ozone. In particular it is desirable that the formulations are substantially free of chlorofluorocarbons especially non hydrogen-containing chlorofluorocarbons such as CCI₃F, CCl₂F₂ and CF₃CCl₃. As used herein "substantially free" means less than 1% w/w based upon the fluorocarbon or hydrogen-containing chlorofluorocarbon propellant, in particular less than 0.5%, for example 0.1% or less.

The propellant may optionally contain an adjuvant having a higher polarity and/or a higher boiling point than the propellant. Polar adjuvants which may be used include (e.g. C₂₋₆) aliphatic alcohols and polyols such as ethanol, isopropanol and propylene glycol, preferably ethanol. In general only small quantities of polar adjuvants (e.g. 0.05 - 3.0% w/w) are required to improve the stability of the dispersion - the use of quantities in excess of 5% w/w may tend to dissolve the medicament. Formulations in accordance with the invention preferably contain less than 1% w/w, e.g. about 0.1% w/w or less, of polar adjuvants. Suitable volatile adjuvants include saturated hydrocarbons such as propane, n-butane, isobutane, pentane and isopentane and alkyl ethers such as dimethyl ether. In general, up to 50% w/w of the propellant may comprise a volatile adjuvant, for example 1 to 30% w/w of a volatile saturated C₁₋₆ hydrocarbon.

However, it is preferable that the formulations of the invention are substantially free of other potential solvating species such as chlorofluorocarbons, ethyl acetate, alkanes, ethers, alcohols and water. In particular, the formulations are substantially free of water, for example containing less than 250ppm, preferably less than 200ppm, more preferably less than 100ppm, for example less than 50ppm water.

A particularly preferred embodiment of the invention provides a pharmaceutical aerosol formulation which consists essentially of anhydrous beclomethasone dipropionate and 1,1,1,2,3,3,3-heptafluoro-n-propane as propellant.

The final aerosol formulation desirably contains 0.005-10% w/w, preferably 0.005-5.0% w/w. especially 0.01-1.0% w/w, for example 0.01-0.5% w/w of beclomethasone dipropionate relative to the total weight of the formulation.

It will be appreciated by those skilled in the art that the aerosol formulations according to the invention may , if desired, contain one or more additional active ingredients. Aerosol compositions containing two active ingredients (in a conventional propellant system) are known, for example, for the treatment of respiratory disorders such as asthma. Accordingly the present invention further provides aerosol formulations in accordance with the invention which contain one or more additional particulate medicaments. Additional medicaments may be selected from any other suitable drug useful in inhalation therapy and which may be presented in a form which is substantially completely insoluble in the selected propellant. Appropriate medicaments may thus be selected from, for example, analgesics, e.g. codeine, dihydromorphine, ergotamine, fentanyl or morphine; anginal preparations, e.g. diltiazem; antiallergics, e.g. cromoglycate, ketotifen or nedocromil; antiinfectives e.g. cephalosporins, penicillins, streptomycin, sulphonamides, tetracyclines and pentamidine; antihistamines, e.g. methapyrilene; anti-inflammatories, e.g. fluticasone, flunisolide, budesonide, tipredane or triamcinolone acetonide; antitussives, e.g. noscapine; bronchodilators, e.g. salmeterol, salbutamol, ephedrine, adrenaline, fenoterol, formoterol, isoprenaline, metaproterenol, phenylephrine, phenylpropanolamine, pirbuterol, reproterol, rimiterol, terbutaline, isoetharine, tulobuterol, orciprenaline, or (-)-4-amino-3,5-dichloro-α-[[[6-[2-(2-pyridinyl)ethoxy]hexyl]-amino]methyl]benzenemethanol; diuretics, e.g. amiloride; anticholinergics e.g. ipratropium, atropine or oxitropium; hormones, e.g. cortisone, hydrocortisone or prednisolone; xanthines e.g. aminophylline, choline theophyllinate, lysine theophyllinate or theophylline; and therapeutic proteins and peptides, e.g. insulin or glucagon. It will be clear to a person skilled in the art that, where appropriate, the medicaments may be used in the form of salts (e.g. as alkali metal or amine salts or as acid addition salts) or as esters (e.g. lower alkyl esters) or as solvates (e.g. hydrates) to optimise the activity and/or stability of the medicament and/or to minimise the solubility of the medicament in the propellant.

Particularly preferred aerosol formulations contain salbutamol (e.g. as the free base or the sulphate salt) or salmeterol (e.g. as the xinafoate salt) in combination with the beclomethasone diproprionate. Combinations of salmeterol xinafoate and beclomethasone dipropionate are preferred.

The formulations of the invention may be prepared by dispersal of the medicament in the selected propellant in an appropriate container, e.g. with the aid of sonication.

Minimising and preferably avoiding the use of formulation excipients e.g. surfactants, cosolvents etc in the aerosol formulations according to the invention is advantageous since the formulations may be substantially taste and odour free, less irritant and less toxic than conventional formulations.

The chemical and physical stability and the pharmaceutical acceptability of the aerosol formulations according to the invention may be determined by techniques well known to those skilled in the art. Thus, for example, the chemical stability of the components may be determined by HPLC assay, for example, after prolonged storage of the product. Physical stability data may be gained from other conventional analytical techniques such as, for example, by leak testing, by valve delivery assay (average shot weights per actuation), by dose reproducibility assay (active ingredient per actuation) and spray distribution analysis.

Canisters generally comprise a container capable of withstanding the vapour pressure of the propellant used such as a metal can of aluminium which may optionally be anodised, lacquer-coated and/or plastic-coated, which container is closed with a metering valve. The metering valves are designed to deliver a metered amount of the formulation per actuation and incorporate a gasket to prevent leakage of propellant through the valve. The gasket may comprise any suitable elastomeric material such as for example low density polyethylene, chlorobutyl, black and white butadiene-acrylonitrile rubbers, butyl rubber and neoprene. Suitable valves are commercially available from manufacturers well known in the aerosol industry, for example, from Valois, France (e.g. DF10, DF30, DF60), Bespak plc, UK (e.g. BK300, BK356, BK357) and 3M-Neotechnic Ltd, UK (e.g. Spraymiser™).

Conventional bulk manufacturing methods and machinery well known to those skilled in the art of pharmaceutical aerosol manufacture may be employed for the preparation of large scale batches for the commercial production of filled canisters. Thus, for example, in one bulk manufacturing method a metering valve is crimped onto an aluminium can to form an empty canister. The particulate medicament is added to a charge vessel and liquified propellant is pressure filled through the charge vessel into a manufacturing vessel. The drug suspension is mixed before recirculation to a filling machine and an aliquot of the drug suspension is then filled through the metering valve into the canister. Typically, in batches prepared for pharmaceutical use, each filled canister is check-weighed, coded with a batch number and packed into a tray for storage before release testing.

Each filled canister is conveniently fitted into a suitable channelling device prior to use to form a metered dose inhaler for administration of the medicament into the lungs or nasal cavity of a patient. Suitable channelling devices comprise for example a valve actuator and a cylindrical or cone-like passage through which medicament may be delivered from the filled canister via the metering valve to the nose or mouth of a patient e.g. a mouthpiece actuator. Metered dose inhalers are designed to deliver a fixed unit dosage of medicament per actuation or "puff", for example in the range of 10 to 5000 microgram medicament per puff.

Administration of medicament may be indicated for the treatment of mild, moderate or severe acute or chronic symptoms or for prophylactic treatment. It will be appreciated that the precise dose administered will depend on the age and condition of the patient, the particular particulate medicament used and the frequency of administration and will ultimately be at the discretion of the attendant physician. When combinations of medicaments are employed the dose of each component of the combination will in general be that employed for each component when used alone. Typically, administration may be one or more times, for example from 1 to 8 times per day, giving for example 1,2,3 or 4 puffs each time.

Suitable daily doses, may be, for example in the range 100 to 2000 microgram of beclomethasone dipropionate, depending on the severity of the disease.

Thus, for example, each valve actuation may deliver 50, 100, 200 or 250 microgram beclomethasone dipropionate. Typically each filled canister for use in a metered dose inhaler contains 100, 160 or 240 metered doses or puffs of medicament.

The metered dose inhalers described herein comprise further aspects of the present invention.

A method of treating respiratory disorders such as, for example, asthma comprises administration by inhalation of an effective amount of a formulation as herein described.

The following non-limitative Example serves to illustrate the invention.

### Example 1

### Aerosol Formulation

Micronised anhydrous beclomethasone dipropionate (60 mg), was weighed into a clean, dry, plastic-coated glass bottle and dry (<50ppm H₂O) 1,1,1,2,3,3,3-heptafluoro-n-propane (18.2g) was added from a vacuum flask. The bottle was quickly sealed with a blank aluminium ferrule. The resulting aerosol contained 0.33% (w/w) beclomethasone dipropionate.

## Claims

1. A canister comprising an aluminium can optionally anodised, lacquer coated and/or plastic coated and closed with a metering valve which contains a pharmaceutical aerosol formulation comprising particulate anhydrous beclomethasone dipropionate and 1,1,1,2,3,3,3-heptafluoro-n-propane as propellant, which formulation is substantially free of surfactant.

2. A canister as claimed in claim 1 which contains 0.005-5.0% w/w of beclomethasone dipropionate relative to the total weight of the formulation.

3. A canister as claimed in claim 1 or claim 2 wherein the particle size of the anhydrous beclomethasone dipropionate is such as to permit inhalation of substantially all of the drug into the lungs upon administration of the aerosol formulation.

4. A canister as claimed in any one of claims 1 to 3 which additionally contains salbutamol.

5. A canister comprising an aluminium can optionally anodised, lacquer coated and/or plastic coated and closed with a metering valve which contains a pharmaceutical aerosol formulation which consists essentially of particulate anhydrous beclomethasone dipropionate and 1,1,1,2,3,3,3-heptafluoro-n-propane as propellant.

6. A metered dose inhaler which comprises a canister as claimed in any one of claims 1 to 5 fitted into a suitable channelling device.

## Patentansprüche

1. Kanister, der eine gegebenenfalls eloxierte, lackierte und/oder kunststoffbeschichtete und mit einem Dosierventil verschlossene Aluminiumdose umfasst, und der eine pharmazeutische Aerosolformulierung enthält, die teilchenförmiges wasserfreies Beclomethasondipropionat und 1,1,1,2,3,3,3-Heptafluor-n-propan als Treibmittel umfasst, wobei die Formulierung im wesentlichen frei von Tensid ist.

2. Kanister gemäss Anspruch 1, der 0,005 bis 5,0 % G/G Beclomethasondipropionat relativ zum Gesamtgewicht der Formulierung enthält.

3. Kanister gemäss Anspruch 1 oder 2, worin die Teilchengrösse des wasserfreien Beclomethasondipropionats derart ist, dass die Inhalation des im wesentlichen gesamten Arzneistoffs in die Lungen bei Verabreichung der Aerosolformulierung erlaubt wird.

4. Kanister gemäss einem der Ansprüche 1 bis 3, der zusätzlich Salbutamol enthält.

5. Kanister, der eine gegebenenfalls eloxierte, lackierte und/oder kunststoffbeschichtete Aluminiumdose umfasst und mit einem Dosierventil verschlossen ist, und der eine pharmazeutische Aerosolformulierung enthält, die im wesentlichen aus teilchenförmigem wasserfreiem Beclomethasondipropionat und 1,1,1,2,3,3,3-Heptafluor-n-propan als Treibmittel besteht.

6. Dosierinhalator, der einen Kanister gemäss einem der Ansprüche 1 bis 5 umfasst, eingepasst in eine geeignete Kanalisierungsvorrichtung.

## Revendications

1. Boîte métallique comprenant une boîte en aluminium éventuellement anodisé, revêtue de laque et/ou revêtue de plastique et fermée avec une valve doseuse, qui contient une formulation d'aérosol pharmaceutique qui comprend du dipropionate de béclométhasone anhydre particulaire et du 1,1,1,2,3,3,3-heptafluoro-n-propane comme gaz propulseur, laquelle formulation est substantiellement dépourvue de tensioactif.

2. Boîte métallique selon la revendication 1, qui contient de 0,005 à 5,0% en poids/poids de dipropionate de béclométhasone par rapport au poids total de la formulation.

3. Boîte métallique selon la revendication 1 ou la revendication 2, dans laquelle la dimension particulaire du dipropionate de béclométhasone anhydre est telle qu'elle permet l'inhalation de substantiellement la totalité du médicament dans les poumons lors de l'administration de la formulation d'aérosol.

4. Boîte métallique selon l'une quelconque des revendications 1 à 3, qui contient de plus du salbutamol.

5. Boîte métallique comprenant une boîte en aluminium éventuellement anodisé, revêtue de laque et/ou revêtue de plastique et fermée avec une valve doseuse, qui contient une formulation d'aérosol pharmaceutique qui consiste essentiellement en dipropionate de béclométhasone anhydre particulaire et en 1,1,1,2,3,3,3-heptafluoro-n-propane comme gaz propulseur.

6. Aérosol-doseur qui comprend une boîte métallique selon l'une quelconque des revendications 1 à 5 ajusté dans un dispositif de canalisation convenable.
